**Europäisches Patentamt**

**European Patent Office**

**Office européen des brevets**

(11) Publication number: **0 065 078**
**B1**

# EUROPEAN PATENT SPECIFICATION

(45) Date of publication of patent specification: **19.06.85**

(21) Application number: **82101719.1**

(22) Date of filing: **05.03.82**

(51) Int. Cl.⁴: **C 07 C 51/50**, C 07 C 57/07, C 07 C 103/133, C 07 C 121/32, C 08 F 2/04

(54) **A process for improving the polymerizability of acrylonitrile, acrylamide, acrylic acid and mixtures thereof.**

(30) Priority: **27.04.81 US 258104**
**27.04.81 US 258235**
**27.04.81 US 258103**

(43) Date of publication of application:
**24.11.82 Bulletin 82/47**

(45) Publication of the grant of the patent:
**19.06.85 Bulletin 85/25**

(84) Designated Contracting States:
**DE FR GB IT NL**

(56) References cited:
**US-A-2 963 459**
**US-A-3 028 426**
**US-A-3 622 533**
**US-A-3 689 558**
**US-A-3 948 867**

(73) Proprietor: **AMERICAN CYANAMID COMPANY**
**1937 West Main Street P.O. Box 60**
**Stamford Connecticut 06904 (US)**

(72) Inventor: **Schmitt, Joseph Michael**
**41 Knollwood Road**
**Ridgefield Connecticut (US)**
Inventor: **Gotthard, George**
**27 Belgrade Terrace**
**West Orange New Jersey 07052 (US)**

(74) Representative: **Wächtershäuser, Günter, Dr.**
**Tal 29**
**D-8000 München 2 (DE)**

Courier Press, Leamington Spa, England.

# 0 065 078

**Description**

This invention relates to treatment of acrylonitrile, acrylamide and related monomers of inferior polymerizability to provide such monomers in a form in which they exhibit improved polymerizability. More particularly, this invention relates to such treatment wherein the treating agent is a borane compound.

Acrylamide is conventionally prepared by the hydration of acrylonitrile as is well-known in the art. This acrylamide generally exits the preparation process as a concentrated aqueous solution, i.e., 30 to 60 weight percent acrylamide. Acrylic acid is conventionally prepared by the oxidation of propylene as is well-known in the art. This material generally is available as concentrated solutions, i.e., 60% to glacial. For economic reasons, it is essential that these products be directly polymerized to water-soluble, high molecular weight products. However, these monomer solutions apparently contain unknown impurities at the level of parts per million, the exact amount and type being undetermined to date. When these monomers are polymerized, even with this very low level of impurities present, quite often totally unacceptable polymers result.

Acrylonitrile, as conventionally prepared, has been found to contain small amounts (parts per million) of aldehyde impurities. A critical impurity has been found to be acrolein. When acrylamide is made from such acrolein-contaminated acrylonitrile and the acrylamide is polymerized to high molecular weight polymers for use as flocculants, the resulting polyacrylamide is oftentimes unsatisfactory in that it contains large amounts of insoluble and/or is not of a sufficient molecular weight for commercial use.

Japanese Application 53-60040 (Publication No. 54-151915 of Mitsui Toatsu discloses the use of a porous-form ion exchange which possesses a primary and/or secondary amine to perform the removal of acrolein.

However, this procedure has not been found to be entirely satisfactory and research continues towards finding other methods for dealing with the problem.

With respect to acrylamide and acrylic acid, in solution polymerization attempts to improve the polymerizability required one or more of the following: (a) very low polymer drying temperatures; (b) extensive and expensive purification of the monomer solution by recrystallization; (c) very long polymerization times; (d) addition of very large amounts of urea or chain transfer agents to the monomer; (e) polymerizing in very dilute solution; and (f) adding post-polymerization stabilizers. Each of these modifications has been found to be unsatisfactory for large-scale commercial use due to being either energy-intensive or expensive in that the rate of production of polymers is drastically reduced or the percent of desirable polymer is reduced to an unacceptable level.

In water-in-oil emulsion polymerizations, attempts at solving the problem entailed: (a) monomer purification by recrystallization; (b) polymerizing very dilute solutions; (c) use of special initiators; (d) addition of urea to the monomer; and/or (e) use of chain transfer agents. However, these modifications have also been found unsatisfactory for the same or similar reasons given above with respect to solution polymerizations.

What is needed, therefore, is an inexpensive and convenient method for improving the polymerizability of poorly polymerizable acrylonitrile, acrylamide, acrylic acid and mixtures thereof so as to avoid the deficiencies noted above. The provision for such a method and its various aspects would fulfill a long-felt need and constitute a significant advance in the art.

In accordance with the present invention, there is provided a process for improving the polymerizability of a monomer of inferior polymerizability selected from acrylonitrile, acrylamide, acrylic acid and mixtures thereof which comprises treating said monomer with an effective amount of a borane compound containing at least one —B—H moiety selected from borohydrides and complexes of boron hydrides for a sufficient time to provide a monomer of improved polymerizability, with the proviso that when the monomer is selected from acrylamide, acrylic acid and mixtures thereof and said monomer contains a sufficient quantity of a metallic ion or compound to form an initiator system in the presence of said borane compound, the amount of inhibitor present in the composition is increased so as to prevent premature polymerization of the composition and when said monomer is substantially free of metallic ion or compound, said monomer is present as a 5 to 20 weight percent solution in water and is free of added inhibitor.

There is further provided a process for producing a water-soluble high molecular weight polymer by polymerizing a monomer of inferior polymerizability selected from acrylamide, acrylic acid, and mixtures thereof, characterized by also conducting the process described immediately above with the proviso that when the monomer is substantially free of metallic ion or compound, the process described above may be performed in one of the following manners, (1) before polymerization is initiated and (2) after polymerization and before drying, which is an added process step in such embodiment, and when the monomer contains a sufficient quantity of a metallic ion or compound to form an initiator system in the presence of said borane compound, the process described immediately above is performed prior to polymerization.

The present invention provides acrylonitrile of greatly reduced acrolein content from an initial acrylonitrile of high acrolein content by simple processing. It also provides acrylamide and acrylic acid of improved polymerizability from initial counterparts of inferior polymerizability. In addition, the present

2

invention provides water-soluble polymers that have higher molecular weights and reduced insolubles when monomers of inferior polymerizability are properly processed according to the invention.

Monomers that can be employed in the various embodiments of the present invention include acrylonitrile, acrylamide, acrylic acid and mixtures thereof.

The borane compounds useful herein are those containing at least one —B—H moiety. Generally, these compounds are catalysts or parts of catalyst systems for the polymerization of acrylonitrile and other monomers in the absence of oxygen, see U.S. Patent 2,963,459. However, the borane compounds are employed in the present invention under conditions which do not lead to premature polymerization. Useful borane compounds include borohydrides and complexes of boron hydrides with other compounds.

Examples of borohydrides useful herein are the borohydrides (tetrahydroborates) of alkali metals, alkaline earth metals as well as those of thorium, mercury, gold, and lead; the cyanoborohydrides of the above metals; Lalancette's Reagent, $Na_2BH_2S_3$; hydridotrialkoxyborates of the above metals; tetramethyl ammonium octahydrotriborate as well as other hydropolyborates, e.g., salts of $B_{12}H_{12}^{-2}$ and the like. Preferable, the alkali metal borohydrides are used. Most preferably, sodium borohydride is used because of its commercial availability.

Examples of complexes of boron hydrides with other compounds include the amine boranes wherein alkylamines are combined with metallic tetrahydroborates. Suitable amines include ammonia, methylamine, dimethylamine, trimethylamine, triethylamine, isopropylamine, diisopropylamine, t-butylamine, N,N-dimethyl-2-methoxyethylamine, pyridine, piperazine, morpholine, methylmorpholine, 2,6-lutidine, methoxypyridine, 4-aminopyridine, and the like.

Alternatively, the borane compound may be supported on a resin and used as a counterion on a quaternary ion exchange resin or as in commercially available Amborane® resins of Rohm and Haas, or amine polymers which are reacted with borohydrides, e.g. poly(4-vinylpyridine)borane.

Any borane compound to be useful herein must, of course, have sufficient stability in the monomer composition to provide the desired benefits described.

In preparing the compositions of the present invention, a suitable borane compound is added to the monomer composition in an effective amount to provide the degree of improvement desired in the monomer of inferior polymerizability. Generally, an amount in the range of 50 parts per million to 2 weight percent based on the weight of the monomer is employed when the borane compound is a borohydride or a boron hydride complex, preferably 100 to 5,000 parts per million.

When the borane compound used is a resin, the monomer should properly contact the resin to ensure that all portions of the liquid monomer effectively contact the resin.

When the monomer employed in the present invention is acrylonitrile, the acrylonitrile is generally free of diluents and metallic ions or compounds and contains acrolein as an impurity and will contain small amounts of a polymerization inhibitor. In preparing a composition comprising acrylonitrile according to the invention, the borane compound and acrylonitrile will comprise the composition without the need for additional ingredients. The amount of inhibitor inherently present in the acrylonitrile as it is produced will prevent premature polymerization even in the presence of the added borane compound.

When the monomer employed in the present invention is selected from acrylamide, acrylic acid and mixtures thereof, two different compositions arise depending upon whether the monomer is substantially free of metallic ions or compounds to form an initiator system in the presence of the borane compound.

In the case when the monomer employed is substantially free of metallic ion or compound, the monomer is used as a 5 to 20 weight percent solution in water and the borane compound is added thereto. No additional usage of inhibitor is necessary and the monomer contains the usual amount of inhibitor associated with the monomer alone, generally entrapped oxygen.

In the case where the monomer contains an amount of metallic ion or compound which forms an effective initiator in the presence of the borohydride compound, it is necessary to increase the amount of inhibitor present so as to prevent premature polymerization, but it is not necessary to dilute the monomer concentration with water although aqueous solutions of the monomer may be used. The most convenient polymerization inhibitor to use is oxygen and it may be provided by means of an air or oxygen sparge. The amount of oxygen to be used is such as to maintain a positive oxygen solubility in the monomer. This should be at least 0.1 part per million, preferably at least 1 part per million and most preferably at least 2 parts per million. Other suitable inhibitors include hydroquinone, monomethyl ether of hydroquinone, catechol, tertiary-butyl catechol, resorcinol, phenylthiazine, eugenol and the like.

When a soluble alkali metal borohydride is used, the pH of the composition preferably should reach the operative pH for the particular alkali metal borohydride used during at least a portion of the time the solution exists. For example, the operative pH for sodium borohydride is about 10.0 and when this pH is reached optimum benefits are achieved. The pH may be obtained by addition of a base such as caustic to the monomer solution or by merely increasing the amount of sodium borohydride employed, this borohydride being itself very basic.

In carrying out the process of improving the polymerizability of a monomer of inferior polymerizability, the solutions prepared as described above are employed. These solutions are treated with the borane compound for an effective time to provide the extent of improved polymerizability desired. Effective times will vary depending upon amount of borane compound used. In the case of acrylonitrile, the time may vary from 5 minutes to 7 days. In the case of acrylamide, acrylic acid and mixtures thereof, the time may vary from 5 minutes to 8 hours.

By "improving the polymerizability of a monomer of inferior polymerizability," as that term is used herein and in the appended claims, is meant providing a monomer which produces a polymer of higher molecular weight, as indicated by standard viscosity, than does the corresponding monomer of inferior polymerizability, produces a polymer containing less insolubles than does the corresponding polymer of inferior polymerizability or produces an acrylonitrile monomer of lower acrolein content than the conventionally prepared acrylonitrile. It also means that when in specific instances the treatment with borane compound is effected after polymerization but prior to drying when drying of the polymer is employed, the resulting dried polymer has higher standard viscosity and lower insolubles than the comparable polymer produced from a monomer of inferior polymerizability which did not receive treatment with a borane compound prior to polymerization or subsequent to polymerization and prior to drying. This latter development is highly surprising and suggests that the detrimental impurities present in the monomers of inferior polymerizability may cause depolymerization and formation of insolubles if not destroyed in time.

After treating the monomer with borane compound for an effective time period, if desired, any excess borane compound may be removed by passing the monomer composition through a column which will attract the borane compound or, preferably, it may be decomposed to the corresponding borate by the addition of an acid. This reaction liberates hydrogen and hence should be done with proper safety precautions. Thus, the use of dilute acids, for relatively extended times, are preferred. Suitable acids include sulfuric, phosphoric, hydrochloric and the like. They should be used at 5 to 20 weight percent solutions with the addition taking at least 15 minutes, preferably 30 minutes to 1 hour. Although shorter times and higher concentrations may be used, there is an increased risk of unwanted, uncontrolled, premature polymerization of the monomer. During the acidification, the hydrogen gas content of the air should be maintained below 4%. Alternatively, an extended treatment time of up to several days without acidification may be allowed for the borane compound to hydrolyze slowly to the corresponding borate.

Polymers and copolymers of acrylamide and acrylic acid or mixtures thereof with other ethylenically unsaturated monomers suitable to produce water-soluble products may be employed using the monomers treated with the borane compound. Such other comonomers include, but are not limited to, methacrylamide, salts of acrylic acid, methacrylic acid, and its salts, methyl acrylate, ethyl acrylate, propyl acrylate, methyl methacrylate, ethyl methacrylate, dimethylaminoethyl acrylate, dimethylaminoethyl methacrylate, hydroxyethyl acrylate, hydroxyethyl methacrylate, dimethylaminoethyl acrylate methosulfate, styrene, acrylonitrile, 3-(methacrylamido)-propyl-trimethyl ammonium chloride, vinyl methyl ether, vinyl ethyl ether, alkali metal or ammonium salts of vinyl sulfonic acid, and the like. All or part of the acrylamide part of the resulting polymers may be hydrolyzed.

The polymerization method to be used may be any one which is conventionally used to polymerize such monomers. This specifically includes solution and emulsion polymerizations, although other techniques such as bead and suspension or dispersion polymerizations may be used. The particular polymerization system for each of these is that which is conventionally used. For solution polymerization this generally entails using one or more azoinitiators, with or without a redox system, and optionally such conventional additives as sequestrants, alcohols and diluents as necessary to the polymerization. For emulsion polymerization, which is a water-in-oil emulsion, this entails using a water-in-oil emulsifying agent, an oil phase such as toluene, xylene, or a paraffinic oil, and a free radical initiator.

As the present invention is independent of the particular polymerization method, further details thereon may readily be found in the prior art. Furthermore, the quantities and the individual componentes will vary according to the monomers polymerized and the process conditions under which the polymerization is to occur.

Even the following borane treatment, it has sometimes been found advantageous to incorporate into the polymerization recipe up to about 20 weight percent, based on the monomer, of urea or a urea-derivative. Generally, 5 to 10 weight percent of urea is used. Suitable compounds of such type are disclosed in U.S. Patent 3,622,533. Alternatively, or in addition, similar amounts of methanol may be used.

In carrying out the polymerization process, it is only necessary that the specified monomers be polymerized alone or in combination with a suitable comonomer in accordance with conventional procedures. If the resulting polymer is to be employed without subsequent drying, the required monomers will be those that have received treatment with the borane compound as described above. If the resulting polymer is to be dried, two alternatives arise: (1) the required monomers may be those that have received treatment with the borane compound as described above or (2) the required monomers may be those which have received no treatment with borane compound in which case the polymer, after completion of polymerization but prior to drying, is treated with borane compound.

As indicated, the advantages of the present invention may also be realized by adding the borane compound, not to the monomer as a treatment, but rather to the resulting polymer gel before drying thereof in instances where such drying is contemplated. This is not as desirable in that there may be great difficulty in uniformly mixing the borane compound into the gel and hydrogen gas evolution in the drier. This shows that, however the borane compound is operating, it seems to work also during drying of a solution polymer.

4

The invention is more fully illustrated in the examples which follow wherein all parts and percentages are by weight unless otherwise specified.

Example 1

To a 4-ounce screw-cap bottle are added 50 grams of an acrylonitrile containing acrolein and the bottle is equipped with a magnetic stirrer. Sodium borohydride is then added in the form and the amounts shown in Table I below. The acrolein content is measured at the beginning of the test and at the times indicated in the table.

The results clearly demonstrate that the sodium borohydride treatment reduces the acrolein content. No polymerization of the acrylonitrile is observed during test operation.

TABLE I
Results of Example 1

| $NaBH_4$ ppm/AN | Added As | Reaction time | Acrolein Before | Acrolein After |
|---|---|---|---|---|
| 50 | 2.5% aqueous | 4 days | 5.2 | 3.4 |
| 50 | 2.5% aqueous | 3.5 days | 24.0 | 6.0 |
| 500 | 2.5% aqueous | 2 days | 24.0 | 1.5 |
| 500 | powder | 4 days | 24.0 | 1.7 |
| 500 | 2.5% aqueous | 10 minutes | 24.0 | 1.2 |
|  |  | 1 hour |  | 0.6 |
|  |  | 3 hours |  | 1.4 |
|  |  | 19 hours |  | 1.4 |
|  |  | 2 days |  | 0.2 |
| 500 | powder | 10 minutes | 24.0 | 1.8 |
|  |  | 1 hour |  | 1.2 |
|  |  | 3 hours |  | 2.7 |
|  |  | 19 hours |  | 3.2 |
|  |  | 2 days |  | 1.5 |

Example 2

Two columns are filled with Amborane® 345 and 355 resins respectively and acrylonitrile (AN) containing 1.3 parts per million (ppm) acrolein is passed through each column under flood conditions. The acrolein content of the exiting acrylonitrile is measured to determine the effectiveness of the treatment. The results are as follows:

| Resin | Acrolein content After 1 bed volume AN | After 10 bed volume AN |
|---|---|---|
| Amborane 345 | .1 | .5 |
| Amborane 355 | .1 | 1.2 |

The results show that the resins remove acrolein but that the treatment capacities of the resins are limited.

Example 3

(a) Acrylonitrile containing about 4 ppm acrolein is employed. In a reaction vessel is placed 125 grams (g) of the acrylonitrile and purged with nitrogen for 20 minutes. Then sodium borohydride powder is added in the amount of 500 ppm (0.0625 g) based on acrylonitrile with the nitrogen purge continuing throughout.

After 15.5 hours no polymerization of the acrylonitrile is observed and the acrolein content is less than 2 ppm.

(b) The procedure of (a) above is repeated except that the borohydride is added in the amount of 2000 ppm (0.25 g). Again no polymerization of the acrylonitrile occurred after the addition is complete and the acrolein content is similarly reduced.

Example 4

(a) Acrylamide 50% aqueous which contains about 25 ppm copper and other conventional impurities found in acrylamide is passed through an ion-exchange column containing a cationic exchange resin (Amberlite® 1R-120 of Rohm and Haas). The material exiting the column contains less than about 0.6 ppm copper.

A portion of the acrylamide processed above is diluted to about 10% by the addition of distilled water and 5000 g thereof are placed in a glass beaker and 0.5 g (1000 ppm) of sodium borohydride powder is added with minimal stirring. Thereafter the mixture remains overnight without stirring and no polymerization is observed.

(b) When the above procedure is repeated using similar acrylamide which has not been passed through an ion-exchange column, the mixture polymerizes within minutes.

Example 5

The procedure of Example 4a is repeated except that the ion-exchanged acrylamide 50% contains 0.7 ppm copper and is not diluted prior to the addition of sodium borohydride. The borohydride is added in increments of about 35 ppm borohydride based upon the monomer allowing about 15 minutes between each increment. About 10 minutes after the borohydride concentration reached 250 ppm, the solution polymerizes.

Example 6
Solution polymerization of monomer of Example 4a

The monomer solution of Example 4a is solution polymerized as follows: a 10.43% aqueous acrylamide solution in the amount of 3160 g is placed in a reaction vessel and magnetically stirred. The following are subsequently added in the amounts indicated while the stirring continued:

16 g anhydrous sodium sulfate,
16 g urea,
4 ml 4.5% aqueous ethylenediaminotetraacetic,
acid.

When solution is complete, the pH of the reaction mass is adjusted to 6.0 with sulfuric acid. A nitrogen purge is started at about 250 ml per minute for 30 minutes while warming the reaction mass to 35°C. With the nitrogen purge continuing, polymerization commences within minutes after the introduction of the catalyst:

500 ppm 2,2'-azobis(2-amidinopropane)dihydrochloride.

The polymerization is allowed to continue substantially adiabatically by insulating the reaction vessel. The polymerizate is allowed to undergo a 23.3°C exotherm over about a 2-hour period. Thereafter the reaction continues for about 18 hours producing about 3000 grams of a stiff gel product.

The gel is subsequently cut into slivers and dried in a convection oven for 4 hours at 85°C to have residual volatiles of 7 to 10%. The dried product is reduced in particle size in a Waring Blender and screened to yield a product at a −20 U.S. mesh particle size.

Example 7
Evaluation of product of Example 6

To evaluate the product prepared in Example 6, the following is done:

The dried product in the amount of 0.3 grams is dissolved in deionized water to produce 300 grams of a 0.1% aqueous polymer solution. The solution is passed through a 100 U.S. mesh weighed screen to filter out any insolubles. The screen is washed with about 500 ml of deionized water at room temperature and dried at 100°C overnight before determination of the amount of insolubles which is reported as percentage of the polymer.

The "as is" viscosity is determined by dissolving 0.3 gram of the product in deionized water over 2 hours to yield a 300 g aqueous solution filtering off the insolubles through a 100 U.S. mesh screen, and then adding enough sodium chloride to form a 1 Molar NaCl solution and determining the Brookfield viscosity thereof using a UL (ultra low) adaptor. This is indicative of the performance of the resultant product in that the higher the number, the more desirable the final product.

The results, along with those for a comparison sample prepared by the same procedure but wherein no sodium borohydride is used, are:

6

# 0 065 078

| | Example 6 | Comparison |
|---|---|---|
| "As is" viscosity, cps | 3.8 | 3.0 |
| % Insolubles | 0.7 | 7.6 |

The results clearly demonstrate that by treating the ion-exchanged acrylamide with sodium borohydride, the "as is" viscosity is greatly increased and the percent insolubles is greatly reduced.

Example 8

The procedure of Example 6 is repeated in a number of runs in which the concentration of sodium borohydride is varied, the reaction time is limited to two hours and the catalyst concentration is reduced to 375 ppm based on the acrylamide. Results of the various runs are as follows:

| $NaBH_4$/AMD ppm | "As Is" Viscosity, cps. | % Insolubles |
|---|---|---|
| 0 | 1.9 | 75.0 |
| 36 | 2.1 | 49.0 |
| 72 | 2.0 | 84.0 |
| 107 | 2.0 | 62.0 |
| 143 | 2.1 | 17.0 |
| 1000 | 3.5 | 0.1 |

Example 9

The procedure of Example 8 is repeated except that the pH of the acrylamide solution is adjusted to 10 prior to the addition of the sodium borohydride. The results are:

| $NaBH_4$/AMD ppm | "As Is" Viscosity, cps. | % Insolubles |
|---|---|---|
| 0 | 1.9 | 75 |
| 100 | 3.7 | — |
| 150 | 3.5 | — |
| 250 | 3.8 | 0.5 |
| 500 | 3.7 | 0.5 |
| 750 | 3.6 | 0.8 |
| 1000 | 3.5 | 0.5 |

The results of Examples 8 and 9 demonstrate that sufficient borohydride must be present at the operative pH range (about 10 for sodium borohydride) to obtain maximum improvements in the polymer. At the lower levels in Example 8 such is not the case. The results also demonstrate that an excess of borohydride is not found to be detrimental.

Example 10

Treatment of ion-exchanged acrylamide with Amborane® resin

Acrylamide 50% aqueous of conventional impurity content is passed through an ion-exchange and as a result has less than 0.5 ppm copper. Thereafter it is diluted to about 10% with deionized water and mixed with varying amounts of Amborane® 355 (a resin containing numerous amine-borane groups) of Rohm and Haas for 2 hours. During this treatment the pH is continually adjusted to be in the range 5.5—6.5. When the various acrylamide samples obtained are polymerized as in Example 6 and evaluated as in Example 7, the treatments and results are as shown in Table II below.

7

# 0 065 078

TABLE II
Results of Example 7

| Acrylamide 50%, g | Amborane® 355, g | "As Is" Viscosity, cps | % Insolubles |
|---|---|---|---|
| 700 | 53 | 3.2 | 0.1 |
| 700 | 10.5 | 3.8 | 0.7 |
| 700 | 7 | 3.3 | 2.6 |
| 700 | 3.5 | 2.9 | 28.0 |
| 700 | — | 2.5 | 38.0 |

Example 11

The procedure of Example 6 is repeated except that the acrylamide had no pretreatment with sodium borohydride. After the polymerization and prior to drying 0.5% sodium borohydride is mixed into one portion of the polyacrylamide and nothing is added to the other portion. After drying, properties are determined as in Example 6 with the following results:

| Polymer portion | "As Is" Viscosity, cps | % Insolubles |
|---|---|---|
| Untreated | 2.6 | 14.6 |
| Treated | 3.5 | 0.6 |

The results clearly show that the borohydride treatment is equally effective whether it be conducted prior to polymerization or merely prior to drying the polymerizate.

Example 12

The basic procedure of Example 6 is repeated except that 10% by weight of the acrylamide is replaced by an equivalent weight of each of the following monomers in separate runs:

(a) Acrylic acid
(b) 2-acrylamido-2-methylpropane sulfonic acid
(c) Dimethylaminoethylmethacrylate methyl sulfate.

For (a) and (b) the pH is adjusted with sodium hydroxide.

Following evaluation according to the procedure of Example 7, the same comparable improved results over similar copolymers prepared from untreated acrylamide of conventional impurities are observed as in Example 7.

Example 13

The procedures of Example 4a and 6 are repeated except that the sodium borohydride is replaced by the following compounds:

Sodium cyanoborohydride,
Potassium borohydride,
Calcium borohydride.

In each instance these borohydrides produced polymers of similar improvements as those shown in Example 7.

Example 14
Treatment of acrylamide with $NaBH_4$

(a) To a glass beaker are added 500 grams of 50% aqueous acrylamide which contained about 25 ppm copper based on the acrylamide and other conventional impurities found in acrylamide. An air sparge is started at the rate of 750 ml/minute and continued during the entire treatment. The pH is raised to 10 by the addition of an appropriate amount of 50% aqueous sodium hydroxide. Thereafter 0.1 g (200 ppm) of sodium borohydride in the form of a 2.5% aqueous solution is added and the mixture is stirred to ensure rapid dispersion of the sodium borohydride. After two hours, the excess sodium borohydride is hydrolyzed to sodium metaborate by the addition of a 5% aqueous $H_3PO_4$ solution over a period of about 30 minutes. No polymerization of the acrylamide is observed during the treatment described.

(b) When the above procedure is repeated in the absence of an air sparge, polymerization of the acrylamide occurs shortly after the addition of the sodium borohydride.

8

# 0 065 078

Example 15
Additional treatments of acrylamide

The procedure of Example 14a is repeated except that in separate runs the following conditions are varied:

(1) the borohydride concentration, (2) the copper content, (3) the amount of air, (4) the hydrolysis conditions, and (5) the pH of the acrylamide when the borohydride is added.

The results are in Table III below wherein it can be seen that with the higher levels of copper present, more air is needed and the hydrolysis must be slower to prevent premature polymerization. Also, at the more rapid hydrolysis conditions, the greater is the likelihood of premature polymerization. In the table, "added rapidly" means within about one minute, "0" means no premature polymerization occurred, and "+" means that there was premature polymerization.

TABLE III
Polymerization during treatment with sodium borohydride

Treating 50% acrylamide solutions

| $NaBH_4$ ppm/AMD | Cu ppm/AMD | Air rate vol/vol sol'n-min. | Hydrolysis condition | Premature polymerization |
|---|---|---|---|---|
| 2000 | 25 | 1.5 | 25% $H_2SO_4$ added rapidly | 0 |
| 1000 | 25 | 1.5 | 25% $H_2SO_4$ added rapidly | 0 |
| 500 | 25 | 1.5 | 25% $H_2SO_4$ added rapidly | 0 |
| 200 | 25 | 1.5 | 25% $H_2SO_4$ added rapidly | + |
| 200 | 25 | 1.5 | 2.5% $H_2SO_4$ added rapidly | + |
| 100 | 25 | 1.5 | 2.5% $H_2SO_4$ added rapidly | + |
| 100 | 25 | 1.5 | 2.5% $H_2SO_4$ added rapidly | 0 |
| 50 | 25 | 1.5 | 25% $H_2SO_4$ added rapidly | 0 |
| 50 | 25 | 1.5 | Solution pH 5.3 when $NaBH_4$ added, immediate polymerization | + |
| 50 | 25 | 1.5 | Solution pH 9 when $NaBH_4$ added, 25% $H_2SO_4$ added rapidly | 0 |

Treating 25% acrylamide solutions

| 200 | 0.1 | 0.07 | 5% $H_3PO_4$ over 30 min. | 0 |
| 200 | 0.1 | 0.07 | 85% $H_3PO_4$ rapidly—no stirring | 0 |
| 200 | 0.1 | 0.07 | 85% $H_3PO_4$ rapidly—with stirring | 0 |
| 200 | 22.0 | 0.07 | 85% $H_3PO_4$ rapidly—with stirring | + |
| 200 | 22.0 | 0.07 | 5% $H_3PO_4$ over 30 min. | + |
| 200 | 2.3 | 0.07 | 5% $H_3PO_4$ over 30 min. | + |
| 200 | 0.5 | 0.07 | 5% $H_3PO_4$ over 30 min. | 0 |
| 200 | 1.0 | 0.07 | 5% $H_3PO_4$ over 30 min. | 0 |
| 200 | 1.9 | 0.07 | 5% $H_3PO_4$ over 30 min. | + |
| 200 | 1.4 | 0.07 | 5% $H_3PO_4$ over 30 min. | 0 |
| 200 | 1.9 | 0.07 | 5% $H_3PO_4$ capillary sparger | 0 |

9

TABLE III (contd.)
Polymerization during treatment with sodium borohydride

| NaBH$_4$ ppm/AMD | Cu ppm/AMD | Treating 50% acrylamide solutions Air rate vol/vol sol'n-min. | Hydrolysis condition | Premature polymerization |
|---|---|---|---|---|
| 200 | 22.0 | 0.07 | 5% H$_3$PO$_4$ capillary sparger | + |
| | | Treating 10% acrylamide solutions | | |
| 200 | 22.0 | 0.03 | 5% H$_3$PO$_4$ in 10 min. | + |
| 200 | 22.0 | 0.06 | 5% H$_3$PO$_4$ in 30 min. | 0 |
| 200 | 3.0 | 0.03 | 5% H$_3$PO$_4$ in 30 min. | 0 |
| 200 | 3.0 | 0.03 | 5% H$_3$PO$_4$ rapidly | 0 |
| 200 | 25.0 | 1.5 | 25% H$_2$SO$_4$ rapidly | 0 |
| 200 | 0.2 | 1.5 | 25% H$_2$SO$_4$ rapidly | 0 |

Example 16
Solution polymerization of monomer of Example 14a

The monomer solution of Example 14a (102.4 g) is solution polymerized as follows: It is placed in a polyethylene reactor and magnetically stirred. The following are subsequently added in the amounts specified while stirring continued:

397.6 g deionized water,
1.3 ml 2% aqueous ethylenediamine tetraacetic acid,
2.1 anhydrous sodium sulfate,
0.51 g anhydrous ammonium sulfate,
2.62 g urea.

When solution is complete, the pH of the reaction mass is adjusted to 4.5 with sulfuric acid. A nitrogen purge is started at about 250 ml/minute for 30 minutes while warming the reaction mass to about 35°C. With the nitrogen purge continuing, polymerization commences within minutes after introducing the catalyst.

0.98 ml 2% aqueous 2,2'-azobis-(2-amidinopropane)dihydrodiloride.

The polymerization is allowed to continue adiabatically by insulating the reactor. The polymerizate is allowed to undergo a 23.2°C exotherm over about a four-hour period prior to being transferred to a curing oven at approximately 65°C where it cures for an additional 18 hours producing about 500 g of a stiff gel product.

The gel is subsequently cut into slivers and portions thereof dried in a convection oven to 7—10% residual volatiles at a temperature of 90°C. The dried product is reduced in particle size in a Waring Blender and screened to yield product at a −20 U.S. mesh particle size.

Example 17
Evaluation of the product of Example 16

To evaluate the product prepared in Example 16, the procedure of Example 6 is followed along with the additional described below:

The percent residual volatiles is determined by measuring the weight loss of a 1 gram sample after drying at 100—110°C overnight.

The extent of hydrolysis (percent carboxyl) is determined by a conductometric tetration of the carboxyl content in the polymer. The lower the number reported, the better is the resulting product for noniomics.

The results along with a comparison polymer prepared by the same procedure but wherein no borane treatment is conducted, are shown in Table IV below. They clearly demonstrate that by pretreating the monomer with 200 ppm sodium borohydride the "as is" viscosity is greatly increased and the percent unsolubles is reduced from about 45% to less than 0.1%.

**0 065 078**

TABLE IV
Results of Example 16 and comparison

|  | Example 16 | Comparison |
|---|---|---|
| "As Is" Viscosity cps | 3.6 | 1.9 |
| % Volatiles | 8.5 | 7.3 |
| % Insolubles | 0.1 | 45.3 |
| % Carboxyl | 0.33 | — |

Example 18

The procedures of Examples 16 and 17 are repeated in separate runs using a different impure acrylamide monomer varying the amount of sodium borohydride and the pH when the borohydride is added. The results are:

| $NaBH_4$/AMD ppm | pH | Time min. | "As Is" Viscosity, cps | % Insolubles |
|---|---|---|---|---|
| 0 | — | — | 1.9 | 45.0 |
| 50 | 10 | 120 | 3.0 | 17.6 |
| 50 | 11 | 120 | 3.2 | 12.9 |
| 100 | 10 | 120 | 3.7 | 2.0 |
| 100 | 11 | 120 | 3.7 | 4.9 |
| 200 | 11 | 120 | 3.6 | 0.1 |

The results demonstrate that as the amount of borohydride is increased, the % insolubles decreases and the viscosity increases. Also, when the amount of borohydride is 200 ppm, a two hour treatment time is sufficient to bring the % insoluble to below 2%.

Example 19

The procedures of Examples 14, 16 and 17 are repeated except that the acrylamide is treated as a 25% solution with 200 ppm $NaBH_4$ at pH 10 for 2 hours. The residual $NaBH_4$ is neutralized with a 5% phosphoric acid solution utilizing a 30 minute addition period to minimize hydrogen content in the reactor exhaust streams.

The results of three separate polymerizations of this treated monomer were:

| Polymerization | "As Is" Viscosity, cps | % Insolubles |
|---|---|---|
| A | 3.8 | 0.8 |
| B | 3.9 | 0.7 |
| C | 4.0 | 0.6 |

Example 20

An acrylamide monomer solution is solution polymerized as follows: 240.4 g of a 49.92% acrylamide solution containing conventional acrylamide impurities are placed in a reactor and mechanically stirred. The following are subsequently added in the amounts indicated while the stirring continued:

199.6 g deionized water,
1.1 g anhydrous ammonium chloride,
6.0 g urea,
3.9 g methanol,
0.9 ml 5% aqueous diethylenetriaminepentaacetic acid.

When solution is complete, the pH of the reaction mass is adjusted to 6.0 with hydrochloric acid. The system is then cooled to about −7°C by a dry ice bath and purged with nitrogen by means of a sparge at about 500 ml per hour for 30 minutes while maintaining the temperature below 0°C. With the nitrogen

11

purge continuing, the reactor is removed from the ice bath and polymerization commences within minutes after the introduction of the catalyst system:

1.1 ml 0.3% aqueous ferrous ammonium sulfate,
4.4 ml 0.1 g ammonium persulfate+7.5 g 2,2'-azobis(2-amidinopropane)dihydrochloride in 100 ml water.

The reactor is capped, the $N_2$ sparge is stopped and the polymerization is allowed to continue substantially adiabatically. The temperature rises to about 72°C over about 95 minutes and the solution is allowed to stand overnight at room temperature producing about 440 g of a stiff gel product.

The gel is subsequently cut into slivers, extruded and dried at 90°C for two hours. The dried product is reduced in particle size in a Waring Blender and screened to yield product at a −20 U.S. mesh particle size.

The above polymerization is performed on an unpurified acrylamide monomer. The procedure is then repeated using sodium borohydride treated acrylamide prepared as in Example 14a as shown in Table V below, wherein the properties of each polymer are shown.

TABLE V

| NaBH$_4$/AMD ppm | Initial pH | Treatment time, min. | "As Is" Viscosity cps. | % Insoluble |
|---|---|---|---|---|
| 0 | — | — | 2.4 | 28.7 |
| 50 | 5.5 | 120 | 2.5 | 28.7 |
| 50 | 9.0 | 120 | 2.4 | 33.5 |
| 200 | 5.5 | 120 | 1.5 | 53.4 |
| 200 | 9.0 | 120 | 4.1 | 1.2 |
| 500 | 5.5 | 120 | 4.0 | 0.6 |
| 500 | 9.0 | 120 | 4.4 | 0.1 |
| 1000 | 5.5 | 10 | 3.9 | 6.2 |
| 1000 | 5.5 | 20 | 4.3 | 0.5 |
| 1000 | 5.5 | 30 | 4.4 | 0.4 |

Example 21

The procedure of Example 20 is repeated except that the amount of sodium borohydride is increased to 2000 ppm and the pH and treatment times are varied. The results are:

| NaBH$_4$/AMD ppm | Initial pH | Time min. | "As Is" Viscosity, cps | % Insolubles |
|---|---|---|---|---|
| 0 | — | — | 2.4 | 28.7 |
| 2000 | 5.3 | 5 | 4.1 | 0.7 |
| 2000 | 8.4 | 5 | 3.4 | 18.1 |
| 2000 | 8.1 | 60 | 4.5 | 1.3 |

Example 22

To determine the long-term stability of polymers prepared after treatment of the monomer with sodium borohydride, samples of monomer are treated under varying conditions and polymerized in accordance with the procedure of Example 20. The properties of the polymers after the specified storage times are given in Table VI. They show excellent storage stability of the polyacrylamide made from the treated monomers.

### TABLE VI
#### Stability of polymer from treated acrylamide

| | | Treating conditions | | "As Is" | |
| Days of storage | Initial pH | NaBH$_4$ ppm | Time hr. | Viscosity cps. | % Insolubles |
| --- | --- | --- | --- | --- | --- |
| — | 5.2 | 2000 | 1.0 | 4.5 | 1.3 |
| 71 | | | | 4.5 | 1.7 |
| 115 | | | | 4.0 | 0.7 |
| — | 5.2 | 1000 | 0.5 | 4.7 | 0.4 |
| 37 | | | | 4.6 | 0.2 |
| 97 | | | | 4.6 | 0.1 |
| — | 9.0 | 500 | 2.0 | 4.6 | 0.1 |
| 36 | | | | 3.9 | 1.9 |
| 58 | | | | 4.2 | 0.8 |
| — | 9.0 | 200 | 2.0 | 4.3 | 1.2 |
| 36 | | | | 4.0 | 3.0 |
| 58 | | | | 4.1 | 2.4 |

Example 23

To determine the long-term stability of monomers which had been treated with sodium borohydride many days prior to polymerization, a sample of acrylamide (impure) is treated with two different amounts of sodium borohydride for different time periods. Thereafter the monomers are stored until they are eventually polymerized in accordance with the procedure of Example 16. The treatment conditions and results are as follows:

| NaBH$_4$/AMD ppm | Initial pH | Time min. | Days stored | "As Is" Viscosity, cps | % Insolubles |
| --- | --- | --- | --- | --- | --- |
| — | — | — | — | 1.9 | 45.0 |
| 1000 | 5.2 | 30 | 53 | 3.7 | 5.5 |
| 2000 | 5.2 | 60 | 93 | 3.7 | 0.3 |

Example 24

Emulsion polymerization of acrylamide

Acrylamide (impure) is treated as in Example 14a with varying amounts of sodium borohydride and varying treatment times as shown in Table VII below. These monomers are then polymerized as a water-in-oil emulsion by the following procedure.

To a suitable reaction vessel are added 378.4 parts acrylamide, as a 51.08% aqueous solution, and 111.6 parts deionized water. To this solution are added 6.9 parts of a 5.6% solution of the disodium salt of ethylenediamine tetraacetic acid and 1 part of a 1.4% aqueous solution of t-butyl hydroperoxide. The pH of the resultant solution is adjusted to 5.0. This constitutes the aqueous monomer phase.

The oil phase is prepared by dissolving 15.0 parts of sorbitan monooleate in 179.2 parts of Exxon LOPS®, a commercially available, clear oily liquid sold by Exxon Chemical.

To a suitable high-speed homogenizer is added the complete oil phase system. The homogenizer is started and the monomer aqueous phase is slowly added thereto to form an emulsion having a viscosity of 1500 centipoises (cps). The dispersed phase of the resultant emulsion has a particle size of about 2.5 microns or less.

To a suitable reaction vessel is added the complete emulsion system with stirring. The resultant medium is purged with nitrogen. Stirring continues and sodium metabisulfite is slowly pumped into the vessel over a period of 6 hours while maintaining the vessel at about 40°C after which about 100 ppm

# 0 065 078

(based on the monomer) have been added. The resultant viscous emulsion exhibits a 99.49% conversion of acrylamide.

Stabilization of the polymer emulsion is accomplished by adding 13.5 parts of a 30% aqueous sodium metabisulfite solution. The emulsion is maintained under polymerizing conditions (60 minutes at 40°C) to substantially completely react the remaining acrylamide. Bisulfite comprises 0.4% of the emulsion which effects stabilization of the polymer system.

To the resultant polymer emulsion is added, as an inverting agent mixture over a period of about 30 minutes, the reaction product of a $C_{12}$—$C_{14}$ alcohol and ethylene oxide (Alfonic 1412-60®) in an amount so as to be 2.1% based upon the total finished emulsion. The resultant emulsion is held at 40°C for an additional hour after which time the product is smooth and particle-free. The dispersed polymer phase has a particle size of 2.5 microns or less.

The "as is" viscosity of the resultant product, as well as those of other products prepared by varying the amount of sodium borohydride and the treatment times are shown in Table VII below. As different samples of impure acrylamide are used for each series, there are three separate controls provided.

## TABLE VII
### Results of emulsion polymerization

| | Treating conditions | | "As Is" |
|---|---|---|---|
| $NaBH_4$/AMD ppm | pH | Time (hrs.) | Viscosity, cps |
| — | — | — | 2.7 |
| 1000 | 10.0 | 2 | 4.4 |
| 2000 | 10.0 | 2 | 4.3 |
| — | — | — | 3.6 |
| 500 | 10.0 | 2 | 4.8 |
| 1000 | 10.0 | 2 | 4.5 |
| 2000 | 10.0 | 2 | 4.5 |
| — | — | — | 4.1 |
| 500 | 10.0 | 4.5 | 5.0 |
| 2000 | 10.0 | 2 | 5.2 |

Example 25
Preparation of copolymers

The basic procedure of Example 16 is repeated except that 10% by weight of the acrylamide is replaced in separate runs by an equivalent weight of each of the following monomers:
  (a) acrylic acid,
  (b) 2-acrylamido-2-methylpropane ammonium sulfonate,
  (c) Dimethylaminoethylmethacrylate methyl sulfate quaternary salt.
  For (a) and (b) the pH is adjusted with ammonium hydroxide.

Comparable improved results over those same copolymers prepared from untreated acrylamide as shown in Example 16 are observed.

Example 26
Use of other borohydrides

The procedures of Examples 14a and 16 are repeated except that the sodium borohydride in separate runs is replaced by the following compounds:

  Sodium cyanoborohydride,
  Potassium borohydride,
  Calcium borohydride.

The improved results obtained with these compounds are similar to those obtained with sodium borohydride.

14

Example 27

The procedure of Example 16 is repeated except that the amount of sodium borohydride is varied in separate runs and urea is added in some runs. All treatments are at pH 10 for 2 hours. The data in Table VIII below demonstrates that when 5% urea is included in the subsequent polymerization recipe, the amount of borohydride needed to treat the monomer is greatly reduced.

TABLE VIII
Results of Example 27

| $NaBH_4$/AMD ppm | Urea | "As Is" Viscosity, cps | % Insolubles |
|---|---|---|---|
| — | Yes | 3.3 | 8.8 |
| 200 | Yes | 4.0 | 0.3 |
| 200 | No | 1.7 | 43.5 |
| 500 | No | 3.2 | 14.8 |
| 1000 | No | 3.4 | 7.0 |
| 2000 | No | 3.6 | 3.5 |
| 2000 | Yes | 3.8 | 0.2 |

Example 28
Hydrolyzed polyacrylamide products

The procedure of Example 16 is repeated with the following variations: (1) the monomer content is increased to about 12%, (2) the pH of the polymerization is raised to 6.0, and (3) the urea and ammonium sulfate are replaced by equal weight of sodium sulfate.

The monomer used had been treated as in Example 14a with 200 ppm sodium borohydride for 2 hours at pH 10.

After the polymeric gel is prepared, it is treated with various amounts of caustic in separate runs for about 15 minutes using optionally additional post-additives to improve its stability. The various runs and results are shown in Table IX below.

TABLE IX
Results of Example 15

| | Control | $NaBH_4$ Treated | Control | $NaBH_4$ Treated | Control | $NaBH_4$ Treated |
|---|---|---|---|---|---|---|
| NaOH level % | 8 | 8 | 30 | 30 | 30 | 30 |
| Post additives, % | | | | | | |
| Sodium metabisulfite | 1.2 | 1.2 | — | — | — | — |
| Sodium thiocyanate | 1.3 | 1.3 | — | — | — | — |
| Thiourea | — | — | — | — | 1.0 | 1.0 |
| "As is" viscosity | 2.1 | 4.5 | 4.3 | 5.4 | 4.9 | 5.3 |
| % Insolubles | WNF | 2.5 | WNF | 0.1 | 3.8 | 0.2 |

WNF means will not filter.

Example 29

An impure sample of acrylic acid substantially free of metallic ions is treated with 1000 ppm of monoisopropylamine borane at pH 7 for 70 hours. The resulting purified acrylic acid monomer is then prepared as an emulsion polymer following the procedure of Example 24. A comparison preparation is also made using the same unpurified acrylic acid. "As is" viscosities of the two emulsions are given below.

| Acrylic acid | "As Is" Viscosity, cps |
| --- | --- |
| Unpurified | 6.0 |
| Purified | 7.1 |

## Claims

1. A process for improving the polymerizability of a monomer comprising:

(a) selecting said monomer from acrylamide, acrylic acid and mixtures thereof, wherein said monomer contains a sufficient quantity of metallic ion or compound to form an initiator system in the presence of the borane compound of (b);

(b) treating said monomer with an effective amount of a borane compound containing at least one —B—H moiety selected from borohydrides and complexes of boron hydride for a sufficient time to provide a monomer of improved polymerizability;

(c) preventing premature polymerization by utilizing an inhibitor selected from a mixture of gases containing oxygen, oxygen, hydroquinone, monomethyl ether of hydroquinone, catechol, tertiary-butyl catechol, resorcinal, phenylthiazine, and eugenol, said inhibitor being continually present throughout the monomer solution for the duration of the process; said process being non-initiating towards polymerization.

2. The process of Claim 1 wherein said inhibitor is air.

3. A process for improving the polymerizability of a monomer comprising:

(a) utilizing a 5 to 20 weight percent solution in water of a monomer which is substantially free of a metallic ion or compound capable of forming an initiator system in the presence of a borane compound of (b), said monomer being selected from acrylonitrile, acrylamide, acrylic acid and mixtures thereof;

(b) treating said monomer with an effective amount of a borane compound containing at least one —B—H moiety selected from borohydrides and complexes of boron hydrides for a sufficient time to provide a monomer of improved polymerizability; said process being non-initiating towards polymerization.

4. The process of Claim 1, 2 or 3 wherein said borane compound is present in an amount of from 50 parts per million to 2 weight percent based on the weight of said monomer.

5. The process of Claim 1 or 2 wherein said treating time is from 5 minutes to 8 hours.

6. The process of Claim 3 wherein said treating time is from 5 minutes to 7 days.

7. The process of Claim 5 further including the added step of decomposing any excess borane compound remaining after said monomer has been improved in polymerizability.

8. The process of Claim 7 wherein said decomposition is effected by acidification under conditions which do not polymerize said monomer.

9. A process for producing a water-soluble high molecular weight polymer by polymerizing a monomer of inferior polymerizability selected from acrylamide, acrylic acid, and mixtures thereof characterized by also conducting the process of Claim 3 either:

(1) before polymerization is initiated, or

(2) after polymerization and before drying of the resulting polymer.

10. A process for producing a water-soluble high molecular weight polymer by polymerizing a monomer of inferior polymerizability selected from acrylamide, acrylic acid, and mixtures thereof characterized by also conducting the process of Claim 1 prior to polymerization.

11. The process of Claim 9 or 10 wherein at least one additional ethylenically-unsaturated monomer is copolymerized with the monomer to provide a water-soluble product.

## Patentansprüche

1. Verfahren zur Verbesserung der Polymerisierbarkeit eines Monomeren, umfassend:

(a) die Auswahl des Monomeren aus Acrylamid, Acrylsäure und Mischungen derselben, wobei das Monomere eine ausreichende Menge an Metall-Ion oder -Verbindung zur Ausbildung eines Initiatorsystems in Gegenwart der Boranverbindung von (b) enthält;

(b) Behandlung des Monomeren mit einer wirksamen Menge einer Boranverbindung, enthaltend mindestens eine —B—H-Einheit, ausgewählt aus Borhydriden und Komplexen des Borhydrids während einer ausreichenden Zeit zur Schaffung eines Monomeren mit verbesserter Polymerisierbarkeit;

(c) Verhinderung einer vorzeitigen Polymerisation durch Nutzung eines Inhibitors, ausgewählt aus einem Gemisch von sauerstoffhaltigen Gasen, Sauerstoff, Hydrochinon, Monomethylether von Hydrochinon, Katechin, Tertiärybutylkatechin, Resorcinal, Phenylthiazin und Eugenol, wobei der Inhibitor während der Dauer des Verfahrens ständig in der Monomerlösung zugegen ist; wobei durch das Verfahren die Polymerisation nicht gestartet wird.

2. Verfahren nach Anspruch 1, wobei der Inhibitor Luft ist.

3. Verfahren zur Verbesserung der Polymerisierbarkeit eines Monomere, umfassend:

16

(a) die Nutzung einer 5 bis 20 Gew.-%igen Lösung in Wasser von einem Monomeren, welches im wesentlichen kein Metall-Ion oder -Verbindung mit der Fähigkeit zur Ausbildung eines Initiatorsystems in Gegenwart einer Boranverbindung von (b) enthält, wobei das Monomere ausgewählt ist aus Acrylnitril, Acrylamid, Acrylsäure und Mischungen derselben;

(b) Behandlung des Monomeren mit einer wirksamen Menge einer Boranverbindung, enthaltend mindestens eine —B—H-Einheit, ausgewählt aus Borhydriden und Komplexen von Borhydriden während einer ausreichenden Zeit zur Schaffung eines Monomeren mit verbesserter Polymerisierbarkeit; wobei das Verfahren die Polymerisation nicht startet.

4. Verfahren nach Anspruch 1, 2 oder 3, wobei die Boranverbindung in einer Menge von 50 Teilen/Million bis 2 Gew.-%, bezogen auf das Gewicht des Monomeren, anwesend ist.

5. Verfahren nach Anspruch 1 oder 2, wobei die Behandlungszeit von 5 Minuten bis 8 Stunden beträgt.

6. Verfahren nach Anspruch 3, wobei die Behandlungszeit von 5 Minuten bis 7 Tage beträgt.

7. Verfahren nach Anspruch 5, umfassend ferner die weitere Verfahrensstufe der Zersetzung jeglicher überschüssiger Boranverbindung, die zurückbleibt, nachdem das Monomere in seiner Polymerisierbarkeit verbessert wurde.

8. Verfahren nach Anspruch 7, wobei die Zersetzung durchgeführt wird durch Ansäuern unter· Bedingungen, bei denen das Monomere nicht polymerisiert.

9. Verfahren zur Herstellung eines wasserlöslichen hoch-molekulargewichtigen Polymeren durch Polymerisation eines Monomeren mit schlechter Polymerisierbarkeit, ausgewählt aus Acrylamid, Acrylsäure und Mischungen derselben, dadurch gekennzeichnet, daß man das Verfahren gemäß Anspruch 3 ebenfalls durchführt, und zwar entweder

(1) bevor die Polymerisation gestartet wird, oder

(2) nach der Polymerisation und vor dem Trocknen des resultierenden Polymeren.

10. Verfahren zur Herstellung eines wasserlöslichen, hoch molekulargewichtigen Polymeren durch Polymerisation eines Monomeren mit schlechter Polymerisierbarkeit, ausgewählt aus Acrylamid, Acrylsäure und Mischungen derselben, dadurch gekennzeichnet, daß man auch das Verfahren gemäß Anspruch 1 vor der Polymerisation durchführt.

11. Verfahren gemäß Anspruch 9 oder 10, wobei mindestens ein weiteres ethylenisch ungesättigtes Monomeres mit dem Monomeren copolymerisiert wird zur Schaffung eines wasserlöslichen Produktes.

## Revendications

1. Un procédé pour améliorer l'aptitude à la polymérisation d'un monomère consistant à:

(a) choisir ledit monomère parmi l'acrylamide, l'acide acrylique et leurs mélanges, ledit monomère contenant une quantité d'un ion ou composé métallique suffisante pour former un système initiateur en présence du composé de type borane de (b);

(b) traiter ledit monomère avec une quantité efficace d'un composé de type borane contenant au moins un fragment —B—H choisi parmi les borohydrures et les complexes d'hydrure de bore pendant une durée suffisante pour obtenir un monomère d'aptitude améliorée à la polymérisation;

(c) empêcher la polymérisation prématurée par l'emploi d'un inhibiteur choisi parmi un mélange de gaz contenant de l'oxygène, l'oxygène, l'hydroquinone, l'éther monométhylique de l'hydroquinone, le pyrocatéchol, le tert-butyl-pyrocatéchol, le résorcinol, la phénylthiazine et l'eugénol, ledit inhibiteur étant toujours présent dans la solution de monomère pendant la durée du procédé; ledit procédé n'étant pas initié à l'égard de la polymérisation.

2. Le procédé de la revendication 1 dans lequel ledit inhibiteur est l'air.

3. Un procédé pour améliorer l'aptitude à la polymérisation d'un monomère, consistant à:

(a) utiliser une solution aqueuse à 5 à 20% en poids d'un monomère qui est essentiellement dépourvu d'ions ou de composé métallique capables de former un système initiateur en présence d'un composé de type borane de (b), ledit monomère étant choisi parmi l'acrylonitrile, l'acrylamide, l'acide acrylique et leurs mélanges;

(b) traiter ledit monomère avec une quantité efficace d'un composé de type borane contenant au moins un fragment —B—H choisi parmi les borohydrures et les complexes d'hydrures de bore pendant une période suffisante pour fournir un monomère d'aptitude améliorée à la polymérisation; ledit procédé n'étant pas initié à l'égard de la polymérisation.

4. Le procédé de la revendication 1, 2 ou 3, dans lequel ledit composé de type borane est présent en une quantité de 50 parties par million à 2% en poids par rapport au poids dudit monomère.

5. Le procédé de la revendication 1 ou 2, dans lequel ladite durée de traitement est de 5 minutes à 8 heures.

6. Le procédé de la revendication 3, dans lequel ladite durée de traitement est de 5 minutes à 7 jours.

7. Le procédé de la revendication 5, comprenant de plus le stade additionnel de décomposition de tout excès de composé de type borane demeurant après que l'aptitude à la polymérisation dudit monomère ait été améliorée.

8. Le procédé de la revendication 7, dans lequel ladite décomposition est effectuée par acidification dans des conditions qui ne polymérisent pas ledit monomère.

9. Un procédé pour produire un polymère soluble dans l'eau de poids moléculaire élevé par

**0 065 078**

polymérisation d'un monomère de faible aptitude à la polymérisation choisi parmi l'acrylamide, l'acide acrylique et leurs mélanges, caractérisé en ce qu'on effectue également le procédé de la revendication 3 soit:

(1) avant l'initiation de la polymérisation, ou

(2) après la polymérisation et avant le séchage du polymère obtenu.

10. Un procédé pour produire un polymère soluble dans l'eau de poids moléculaire élevé par polymérisation d'un monomère de faible aptitude à la polymérisation choisi parmi l'acrylamide, l'acide acrylique et leurs mélanges, caractérisé en ce qu'on effectue également le procédé de la revendication 1 avant la polymérisation.

11. Le procédé de la revendication 9 ou 10, dans lequel au moins un monomère additionnel à insaturation éthylénique est copolymérisé avec le monomère pour fournir un produit soluble dans l'eau.